(19) Europäisches Patentamt
European Patent Office
Office européen des brevets

(11) **EP 3 871 765 A1**

(12) **EUROPEAN PATENT APPLICATION**

(43) Date of publication:
**01.09.2021 Bulletin 2021/35**

(21) Application number: **20305188.3**

(22) Date of filing: **26.02.2020**

(51) Int Cl.:
*B01J 13/18* *(2006.01)*      *A61K 8/11* *(2006.01)*
*A61Q 19/10* *(2006.01)*     *C11D 3/50* *(2006.01)*
*C11D 17/00* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Takasago International Corporation Tokyo 144-8721 (JP)**

(72) Inventors:
• **BETT, William**
  **Paris (FR)**
• **RIBAUT, Tiphaine**
  **Paris (FR)**

(74) Representative: **Cabinet Beau de Loménie**
  **158, rue de l'Université**
  **75340 Paris Cedex 07 (FR)**

(54) **AQUEOUS DISPERSION OF MICROCAPSULES, AND USES THEREOF**

(57) The invention relates to an aqueous dispersion of microcapsules, said microcapsules comprising a hydrophobic core and a shell, wherein said shell is formed of the reaction product of (i) at least one $\alpha,\beta$-unsaturated carbonyl compound, and (ii) at least one multifunctional thiol compound. The invention also relates to a process for the manufacture of such an aqueous dispersion.

EP 3 871 765 A1

## Description

### Field of the Invention

The present invention relates to an aqueous dispersion of microcapsules which comprise a hydrophobic core enclosed within a shell, a process for the manufacture of an aqueous dispersion of microcapsules, as well as consumer products (such as laundry products, personal care products and cosmetic products) containing that aqueous dispersion of microcapsules.

### Background

There is continuing interest in the preparation of fragrance compositions and in the use of such compositions in consumer products. Consumers are in growing demand for perfumed products, which provide a perception of e.g. freshness and/or stimulation, which in turn reinforces consumer confidence in the efficacy of such products. Fragrance compositions can be incorporated into end products in free form and/or in encapsulated form. Microencapsulation represents a common solution to protect (e.g. upon storage) and control the delivery of hydrophobic materials such as fragrances. General descriptions and methods of preparation of microcapsules can be found in "MICROENCAPSULATION: Methods and Industrial Applications Edited by Benita and Simon (Marcel Dekker, Inc. 1996)". Microcapsules are also described in Kirk Othmer's Encyclopaedia of Chemical Technology 5th edition. Microcapsules can be formed by a variety of techniques. Mechanically formed capsules can be formed by means, such as spray chilling, by compression of solids or by spray drying emulsions. Chemically formed capsules are produced by chemical reactions forming ionic or covalent bonds using techniques such as co-acervation, interfacial polymerisation, condensation reactions and free radical polymerisation. One type of microcapsule, referred to as a wall or shell or core-shell microcapsule, comprises a generally spherical shell of water- and oil-insoluble materials, typically a network polymer material, within which fragrance or other hydrophobic material is contained.

Over the past few years there has also been a growing demand for environmentally friendly consumer products. In this context, the Applicant has developed fragrance-containing microcapsules which display satisfactory olfactory properties when incorporated into consumer products, and which are biodegradable.

### Summary of the invention

The present invention relates to an aqueous dispersion of microcapsules, said microcapsules comprising a hydrophobic core and a shell, wherein said shell is formed of the reaction product of (i) at least one $\alpha,\beta$-unsaturated carbonyl compound, and (ii) at least one multifunctional thiol compound. The invention also relates to a consumer product comprising the above-mentioned aqueous dispersion, and to a process for the manufacture of such an aqueous dispersion.

### Description of the invention

Unless otherwise stated, all percentages are weight percentages.

Unless otherwise indicated, all chemical terms have the meanings defined by the IUPAC Compendium of Chemical Terminology, 2nd Edition compiled by A. D. McNaught and A. Wilkinson Blackwell Scientific Publications Oxford 1997, and IUPAC Nomenclature of Organic Chemistry, published by Blackwell Scientific Publications Oxford 1993 ISBN 0632034882.

Unless otherwise indicated, "(meth)acrylate" (or "(meth)acrylic") means methacrylate (or methacrylic) and/or acrylate (or acrylic). For example, it means methacrylate (or methacrylic). For example it means acrylate (or acrylic). For example, it means methacrylate (or methacrylic) and acrylate (or acrylic).

Unless otherwise indicated, room temperature is from 20 to 25°C, and preferably 20°C.

Certain substances, notably perfumery molecules, may exist as distinct isomers (or as mixture of distinct isomers). Hereinafter, they may be identified also by means of their CAS number. In these cases, the CAS number of a single isomer is reported. However, and unless otherwise indicated, the reference shall be understood to cover all existing isomers.

In the context of the present invention, the terms "dispersion" and "slurry" can be used interchangeably.

In the context of the present invention, the adjective "multifunctional", when applied to a compound, indicates that the compound comprises at least two identical reactive functions (such as thiol, (meth)acrylate, (meth)acrylamide).

In the context of the present invention, the various embodiments described in the various aspects of the invention can be combined.

In a first aspect, the present invention relates to an aqueous dispersion of microcapsules, said microcapsules comprising a hydrophobic core and a shell, wherein said shell is formed of the reaction product of (i) at least one $\alpha,\beta$-unsaturated carbonyl compound, and (ii) at least one multifunctional thiol compound.

$\alpha,\beta$-unsaturated carbonyl compounds (i) useful to form the shell of the microcapsules are any suitable compounds comprising a carbonyl group and at least another (electron-withdrawing) group capable of reacting with a thiol group of the multifunctional thiol compound.

In one embodiment, the $\alpha,\beta$-unsaturated carbonyl compound (i) used to form the shell of the microcapsules is an anhydride or a multifunctional (meth)acrylamide. Suitable anhydrides include methacrylic anhydride and acrylic anhydride. Suitable multifunctional (meth)acrylamides include N,N'-methylenebisacrylamide and N,N'-methylenebismethacrylamide.

In another embodiment, the $\alpha,\beta$-unsaturated carbonyl compound (i) used to form the shell of the microcapsules is a multifunctional (meth)acrylate. Suitable multifunctional (meth)acrylates include di(meth)acrylates, tri(meth)acrylates, tetra(meth)acrylates, penta(meth)acrylates, and hexa(meth)acrylates. In a preferred embodiment, the multifunctional (meth)acrylate is an ester of (meth)acrylic acid with a linear or branched, alicyclic, aromatic or heterocyclic $(C_2-C_{24})$alcohol, preferably a $(C_2-C_{12})$alcohol, or an ester of (meth)acrylic acid with a $(C_2-C_{24})$polyethylene glycol, preferably a $(C_2-C_{12})$polyethylene glycol. Suitable alcohols include those having a number average molecular weight of up to about 6,000. Suitable polyethylene glycols include those having a number average molecular weight of up to about 7,500.

In yet a preferred embodiment, the multifunctional (meth)acrylate is selected from 1,4-butylene glycol diacrylate, ethylene glycol diacrylate, diethylene glycol diacrylate, triethylene glycol diacrylate, neopentylglycol diacrylate, triglycerol diacrylate, 1,6-hexane diol diacrylate, trimethylolpropane triacrylate, trimethylolpropane ethoxylate triacrylate, trimethylolpropane propoxylate triacrylate, pentaerythritol triacrylate, tris-2-hydroxyethyl isocyanurate triacrylate, glycerol triacrylate, pentaerythritol tetraacrylate, ditrimethylolpropane tetraacrylate, dipentaerythritol pentaacrylate, dipentaerythritol hexaacrylate, 1,3-butylene glycol dimethacrylate, 1,4-butylene glycol dimethacrylate, 1,2-propylene glycol dimethacrylate, 1,3-propylene glycol dimethacrylate, ethylene glycol dimethacrylate, diethylene glycol dimethacrylate, glycerol dimethacrylate, 1,6-hexane diol dimethacrylate, bisphenol A dimethacrylate, bisphenol A ethoxylate dimethacrylate, pentaerythritol trimethacrylate, glycerol trimethacrylate, trimethylolpropane trimethacrylate, tris-2-hydroxyethyl isocyanurate trimethacrylate, ethoxylated pentaerythritol tetramethacrylate, tris[2-(acryloyloxy)ethyl]isocyanurate, 2-[4,6-bis(2-propenoyloxyethyl)-1,3,5-triazin-2-yl]ethyl prop-2-enoate, and mixtures thereof.

In yet a preferred embodiment, the multifunctional (meth)acrylate is selected from 1,4-butylene glycol diacrylate, ethylene glycol diacrylate, trimethylolpropane triacrylate, pentaerythritol triacrylate, glycerol triacrylate, pentaerythritol tetraacrylate, ditrimethylolpropane tetraacrylate, 1,4-butylene glycol dimethacrylate, ethylene glycol dimethacrylate, trimethylolpropane trimethacrylate, glycerol trimethacrylate, and mixtures thereof.

The $\alpha,\beta$-unsaturated carbonyl compound (i) may be used in an amount ranging from 20 and 70% by weight, and preferably from 30 and 60% by weight, relative to the total weight of compounds (i) and (ii).

The multifunctional thiol compound (ii) useful to form the shell of the microcapsules is any suitable compounds comprising a plurality of pendant or terminally positioned thiol groups, i.e. SH. The multifunctional thiol compound can be a di-functional thiol (with two thiol groups per compound), a tri-functional thiol (with three thiol groups per compound), a tetra-functional thiol (with four thiol groups per compound), a penta-functional thiol (with five thiol groups per compound), a hexa-functional thiol (with six thiol groups per compound), or a higher functionalized thiol compound (with more than six thiol groups per compound). The multifunctional thiol compound (ii) may be a linear or branched aliphatic thiol or aromatic thiol, or a thio-functionalized aliphatic or aromatic heterocycle. Preferably, the multifunctional thiol compound (ii) is free of disulfide linkages. The thiol groups in the multifunctional thiol compound (ii) may be separated from one another in a given molecule by an aliphatic group, aromatic group, ester, polyester, ether, or polyether groups.

Multifunctional thiol compounds (ii) useful to form the shell of the microcapsules may be ethylene glycol bismercaptoacetate, 1,8-dimercapto-3,6-dioxaoctane, dimercaptodiethyl sulfide, 1,6-hexanedithiol, propane-1,2,3-trithiol, 1,2-bis[(2-mercaptoethyl)thio]-3-mercaptopropane, tetrakis(7-mercapto-2,5-dithiaheptyl]methane, trimethylolpropane tris(2-mercaptoacetate), pentaerythritol tetrakis(2-mercaptoacetate), ethylene glycol bis(3-mercaptopropionate), dipentaerythritol hexakis(3-mercaptopropionate), 1,4-butanediol bis(3-mercaptopropionate), tris[2-(3-mercaptopropionyloxy)ethyl]isocyanurate, tetraethylene glycol bis(3-mercaptopropionate), trimethylolethane trimercaptoacetate, 1,4-butanediol bismercaptoacetate, trithiocyanuric acid, pentaerythritol tetrakis(3-mercaptopropionate), trimethylolpropane tris(3-mercaptopropionate), trimethylolpropane tris(3-mercaptobutyrate), 1,4-butanediol bis(3-mercaptobutyrate), poly-2-mercaptoacetate, benzene-1,2-dithiol, 1,4-butanedithiol, 4,4'-biphenyldithiol, benzene-1,4-dithiol, toluene-3,4-dithiol, 1,4-dithiothreitol, 1,3,4-thiadiazole-2,5-dithiol, 1,3,5-benzenetrithiol, 4,4'-bis(mercaptomethylbiphenyl), 1,3,5-tris(3-mercaptobutyryloxyethyl)-1,3,5-triazine-2,4,6(1H,3H,5H)-trione, 3,7-dithia-1,9-nonanedithiol, and mixtures thereof, which are all commercially available or synthesizable by methods known in the art.

In a preferred embodiment, the multifunctional thiol compound (ii) is selected from ethylene glycol bismercaptoacetate, trimethylolpropane tris(3-mercaptopropionate), pentaerythritol tetrakis(3-mercaptopropionate), 1,8-dimercapto-3,6-dioxaoctane, and mixtures thereof.

In a particularly preferred embodiment, the multifunctional thiol compound (ii) is selected from ethylene glycol bismercaptoacetate, trimethylolpropane tris(3-mercaptopropionate), pentaerythritol tetrakis(3-mercaptopropionate), and mixtures thereof.

In a preferred embodiment, the aqueous dispersion of the invention does not comprise any compound in category (i) or (ii) having a single $\alpha,\beta$-unsaturated carbonyl group as compound (i) or a single thiol group as compound (ii).

The multifunctional thiol compounds (ii) may be used in an amount ranging from 30 and 80% by weight, and preferably from 40 and 70% by weight, relative to the total weight of compounds (i) and (ii).

At least one catalyst (iii) may be added for accelerating the reaction between the (i) at least one $\alpha,\beta$-unsaturated carbonyl compound and the (ii) at least one multifunctional thiol compound.

In one embodiment, the catalyst (iii) is selected from ammonia or amine compounds, in particular primary and tertiary amine compounds, nitrogen catalysts others than amine compounds such as amidine compounds, guanidine or arginine, organophosphorous compounds, and water-soluble alkali metal carbonates and hydroxides. The catalyst (iii) is preferably selected from hexylamine, octylamine, pentylamine, diethylamine, triethylamine, diethylene triamine, tetraethylenepentamine, triethylene tetramine, pentaethylene hexamine, lysine, 3-aminopropyl triethoxysilane, 2-amino-2-methyl-1-propanol, 1,8-diazabicyclo[5.4.0]undec-7-ene (DBU), 1,5-diazabicyclo[4.3.0]non-5-ene (DBN), 1,4-diazabicyclo[2.2.2]octane (DABCO), N,N-dimethylbenzylamine, dimethylphenylphosphine, and mixtures thereof.

In a preferred embodiment, the catalyst (iii) is an amine compound selected from primary n-($C_2$-$C_{16}$)monoalkylamine compounds or tertiary amine compounds in $C_2$-$C_{24}$. In a particularly preferred embodiment, the catalyst (iii) is a primary n-($C_2$-$C_{16}$)monoalkylamine compound selected from hexylamine, octylamine, pentylamine, and mixtures thereof. Hexylamine is the preferred catalyst (iii).

The catalyst (iii) may be present in an amount ranging from 0.1 to 5% by weight, and preferably from 0.5 to 3% by weight, relative to the total weight of compounds (i) and (ii).

The catalyst (iii) may be present in a molar amount ranging from 0.2 to 6% by mole, and preferably from 0.5 to 4% by mole, relative to the total molar amounts of compounds (i) and (ii).

The dispersion may comprise, for example essentially consists of, a water-based liquid medium (i.e. the dispersing medium) and a plurality of microcapsules dispersed in the medium. Traces of other ingredients used in the manufacturing process (such as unreacted monomers) may also be present.

In one embodiment, the water-based liquid medium comprises, for example essentially consists of, water, such as deionized water.

In one embodiment, the hydrophobic core of the microcapsules comprises such as essentially consists of, such as consists of, a fragrance, which in that case typically represents from 20% to 45%, such as at least 25%, for example at least 30% or at least 33%, and such as no more than 40%, for example no more than 35%, by weight relative to the total weight of the aqueous dispersion.

In one embodiment, the microcapsules as presently disclosed have a shell thickness from about 80 nm to about 800 nm, such as from about 150 nm to about 700 nm, for example from about 180 nm to about 500 nm, when dried and opened and observed with a scanning electron microscope.

In one embodiment, when the hydrophobic core of the microcapsules as presently disclosed comprise a fragrance, the fragrance to shell weight ratio is from about 50:1 to about 1:1, such as from about 30:1 to about 1:1, or from about 20:1 to about 1:1, for example from about 10:1 to about 1:1.

In one embodiment, the microcapsules presently disclosed are substantially spherical.

In one embodiment, the microcapsules presently disclosed have a median volume diameter (D(v;0.5) value) equal to or greater than 1 micrometer (1 $\mu$m), for example equal to or greater than 7.5 micrometers (7.5 $\mu$m), or equal to or greater than 10 $\mu$m, such as equal to or greater than 15 $\mu$m, or equal to or greater than 20 $\mu$m, for example equal to or greater than 25 $\mu$m. In a further embodiment, the microcapsules presently disclosed have a median volume diameter equal to or less than 250 micrometers (250 $\mu$m), or equal to or less than 100 micrometers (100 $\mu$m), or equal to or less than 65 micrometers (65 $\mu$m), for example equal to or less than 50 $\mu$m, such as equal to or less than 45 $\mu$m, for example equal to or less than 40 $\mu$m. In a further embodiment, the microcapsules presently disclosed have a median volume diameter from 1 micrometer (1 $\mu$m) to 250 micrometers (250 $\mu$m), or from 1 micrometer (1 $\mu$m) to 100 micrometers (100 $\mu$m), or from 7.5 micrometers (7.5 $\mu$m) to 65 micrometers (65 $\mu$m), or from 7.5 $\mu$m to 50 $\mu$m, or from 10 $\mu$m to 50 $\mu$m, or from 7.5 $\mu$m to 45 $\mu$m, or from 10 $\mu$m to 45 $\mu$m, or from 15 $\mu$m to 45 $\mu$m, or from 15 $\mu$m to 40 $\mu$m, or from 20 $\mu$m to 45 $\mu$m, or from 25 $\mu$m to 45 $\mu$m, or from 25 $\mu$m to 40 $\mu$m, or from 25 $\mu$m to 35 $\mu$m.

The microcapsule median volume diameter is measured by light scattering using for example a Horiba® or a Malvern® Laser scattering particle Size Distribution Analyzer or an equivalent instrument working on the principle of Low Angle Laser Light Scattering (LALLS) following the general guidelines set out in ISO 13320 "Particle Size Analysis - Laser Diffraction Methods".

As indicated above the hydrophobic core of the microcapsules can comprise a fragrance. A fragrance comprises at least one, and preferably a mixture of two or more olfactively active (i.e. odoriferous) compounds typically but not necessarily providing a pleasant smell. The fragrance thus typically comprises at least one, such as et least two, such as at least five, or at least eight distinct fragrance compounds. It can comprise highly complex mixtures of fragrance compounds, chosen to provide any desired odour. In the context of the present invention the term "fragrance" is intended to be synonymous with "perfume". Fragrance compounds typically used in the field of perfumery and suitable for the purposes of the present invention are described more fully in S. Arctander, Perfume Flavors and Chemicals 1969, Vols. I and II, Montclair, N. J. and in The Merck Index, 8th edition, Merck & Co., Inc. Rahway, N. J. The term "fragrance compound" encompasses naturally occurring as well as synthetic materials known for use in perfumes, as well as animal oils. A fragrance compound can also be any natural oil or extract used in a fragrance composition. Natural oils and extracts are described in The Essential Oils by E. Guenther published in 1949 by D. Van Nostrand Company, and may include extracts, pressings, collection of exudates, and distillates from any part of suitable plants: roots, rhizomes, bulbs, corms, stem, bark, heartwood, leaves, flowers, seeds and fruit. Examples of such extracts and distillates include citrus fruit oils such as orange, mandarin, grapefruit, lime or lemon oils, tree oils such as pine, or cedar wood, herb oils such as peppermint, thyme, lavender, basil, rosemary, clove or flower extracts such as rose, jasmine, muguet, or geranium oil.

In one embodiment, each fragrance compound has a molecular weight greater than 100 g.mol$^{-1}$, preferably greater than 120 g.mol$^{-1}$ and lower than 325 g.mol$^{-1}$, preferably lower than 300 g.mol$^{-1}$. In a further embodiment each fragrance compound has a boiling point in the range 80-400°C, such as in the range 100-350°C, when measured at 760 mm Hg.

In a preferred embodiment, the fragrance compounds can advantageously be selected from the following list:

- $C_8$-$C_{18}$ hydrocarbons, preferably delta-3-carene, alpha-pinene, beta-pinene, alpha-terpinene, gamma-terpinene, p-cymene, bisabolene, camphene, caryophyllene, cedrene, farnesene, limonene, longifolene, myrcene, ocimene, valencene, (E,Z)-1,3,5-undecatriene;
- $C_2$-$C_{18}$ aliphatic alcohols, preferably hexanol, octanol, 3-octanol, 2,6-dimethylheptanol, 2-methylheptanol, 2-methyloctanol, (E)-3-hexenol, (E)- and (Z)-3-hexenol, 1-octen-3-ol, mixtures of 3,4,5,6,6-pentamethyl-3/4-hepten-2-ol and 3,5,6,6-tetramethyl-4-methyleneheptan-2-ol, (E,Z)-2,6-nonadienol, 3,7-dimethyl-7-methoxyoctan-2-ol, 9-decenol, 10-undecenol, 4-methyl-3-decen-5-ol;
- $C_2$-$C_{18}$ aliphatic aldehydes and their acetals, preferably hexanal, heptanal, octanal, nonanal, decanal, undecanal, dodecanal, tridecanal, 2-methyloctanal, 2-methylnonanal, (E)-2-hexenal, (Z)-4-heptenal, 2,6-dimethyl-5-heptenal, 10-undecenal, (E)-4-decenal, 2-dodecenal, 2,6,10-trimethyl-5,9-undecadienal, heptanal diethyl acetal, 1,1-dimethoxy-2,2,5-trimethyl-4-hexene, citronellyl oxyacetaldehyde;
- $C_3$-$C_{18}$ aliphatic ketones and oximes thereof, preferably 2-heptanone, 2-octanone, 3-octanone, 2-nonanone, 5-methyl-3-heptanone, 5-methyl-3-heptanone oxime, 2,4,4,7-tetramethyl-6-octen-3-one;
- $C_2$-$C_{18}$ aliphatic sulphur-containing compounds, preferably 3-methylthiohexanol, 3-methylthiohexyl acetate, 3-mercaptohexanol, 3-mercaptohexyl acetate, 3-mercaptohexyl butyrate, 3-acetylthiohexyl acetate, 1-menthene-8-thiol;
- $C_2$-$C_{18}$ aliphatic nitrile-containing compounds, preferably 2-nonenenitrile, 2-tridecenenenitrile, 2,12-tridecenenenitrile, 3,7-dimethyl-2,6-octadienenitrile, 3,7-dimethyl-6-octenenitrile;
- $C_2$-$C_{18}$ aliphatic carboxylic acids and esters thereof, preferably (E)- and (Z)-3-hexenyl formate, ethyl acetoacetate, isoamyl acetate, hexyl acetate, 3,5,5-trimethylhexyl acetate, 3-methyl-2-butenyl acetate, (E)-2-hexenyl acetate, (E)- and (Z)-3-hexenyl acetate, octyl acetate, 3-octyl acetate, 1-octen-3-yl acetate, ethyl butyrate, butyl butyrate, isoamyl butyrate, hexyl butyrate, (E)- and (Z)-3-hexenyl isobutyrate, hexyl crotonate, ethyl isovalerate, ethyl 2-methylpentanoate, ethyl hexanoate, allyl hexanoate, ethyl heptanoate, allyl heptanoate, ethyl octanoate, ethyl (E,Z)-2,4-decadienoate, methyl 2-octynoate, methyl 2-nonynoate, allyl-2-isoamyloxyacetate, methyl-3,7-dimethyl-2,6-octadienoate;
- $C_4$-$C_{18}$ acyclic terpene alcohols, preferably citronellol, geraniol, nerol, linalool, lavandulol, nerolidol, farnesol, tetrahydrolinalool, tetrahydrogeraniol, 2,6-dimethyl-7-octen-2-ol, 2,6-dimethyloctan-2-ol, 2-methyl-6-methylene-7-octen-2-ol, 2,6-dimethyl-5,7-octadien-2-ol, 2,6-dimethyl-3,5-octadien-2-ol, 3,7-dimethyl-4,6-octadien-3-ol, 3,7-dimethyl-1,5,7-octatrien-3-ol, 2,6-dimethyl-2,5,7-octatrien-1-ol;
- $C_4$-$C_{18}$ acyclic terpene aldehydes and ketones, preferably geranial, neral, citronellal, 7-hydroxy-3,7-dimethyloctanal, 7-methoxy-3,7-dimethyloctanal, 2,6,10-trimethyl-9-undecenal, geranylacetone, and the dimethyl and diethyl acetals of geranial, neral, 7-hydroxy-3,7-dimethyloctanal;
- $C_4$-$C_{18}$ cyclic terpene alcohols, preferably alpha-terpineol, terpineol-4, menthan-8-ol, menthan-1-ol, menthan-7-ol, borneol, isoborneol, linalool oxide, nopol, cedrol, ambrinol, vetiverol, guaiol;
- $C_4$-$C_{18}$ cyclic terpene aldehydes and ketones, preferably fenchone, alpha-ionone, beta-ionone, alpha-n-methylionone, beta-n-methylionone, alpha-isomethylionone, beta-isomethylionone, alpha-irone, alpha-damascone, beta-damascone, beta-damascenone, delta-damascone, gamma-damascone, 1-(2,4,4-trimethyl-2-cyclohexen-1-yl)-2-buten-1-one, 1,3,4,6,7,8a-hexahydro-1,1,5,5-tetramethyl-2H-2,4a-methanonaphthalen-8(5H)-one, nootkatone, dihydronootkatone, alpha-sinensal, beta-sinensal, methyl cedryl ketone;
- $C_4$-$C_{18}$ cyclic alcohols, preferably 4-tert-butylclyclohexanol, 3,3,5-trimethylcyclohexanol, 3-isocamphylclyclohexanol, 2,6,9-trimethyl-Z2,Z5,E9-cyclododecatrien-1-ol, 2-isobutyl-4-methyltetrahydro-2H-pyran-4-ol;
- $C_4$-$C_{18}$ cycloaliphatic alcohols, preferably alpha-3,3-trimethylcyclohexylmethanol, 2-methyl-4-(2,2,3-trimethyl-3-cyclopent-1-yl)butanol, 2-methyl-4-(2,2,3-trimethyl-3-cyclopent-1-yl)-2-buten-1-ol, 2-ethyl-4-(2,2,3-trimethyl-3-cyclopent-1-yl)-2-buten-1-ol, 3-methyl-5-(2,2,3-trimethyl-3-cyclopent-1-yl)-pentan-2-ol, 3-methyl-5-(2,2,3-trimethyl-3-cyclopent-1-yl)-4-penten-2-ol, 3,3-dimethyl-5-(2,2,3-trimethyl-3-cyclopent-1-yl)-4-penten-2-ol, 1-(2,2,6-trimethylcyclohexyl)pentan-3-ol, 1-(2,2,6-trimethylcyclohexyl)hexan- 3-ol;

- $C_4$-$C_{18}$ cyclic and cycloaliphatic ethers, preferably cedryl methyl ether, cyclododecyl methyl ether, (ethoxymethoxy)cyclododecane, alpha-cedrene epoxide, 3a,6,6,9a-tetramethyl-dodecahydronaphtho[2,1-b]furan, 3a-ethyl-6,6,9a-trimethyldodecahydro-naphtho[2,1-b]furan, 1,5,9-trimethyl-13-oxabicyclo[10.1.0]trideca-4,8-diene, rose oxide, 2-(2,4-dimethyl-3-cyclohexen-1-yl)-5-methyl-5-(1-methylpropyl)-1,3-dioxane;
- $C_4$-$C_{18}$ cyclic ketones, preferably 4-tert-butylcyclohexanone, 2,2,5-trimethyl-5-pentylcyclopentanone, 2-heptylcyclopentanone, 2-pentylcyclopentanone, 2-hydroxy-3-methyl-2-cyclopenten-1-one, 3-methyl-cis-2-penten-1-yl-2-cyclopenten-1-one, 3-methyl-2-pentyl-2-cyclopenten-1-one, 3-methyl-4-cyclopentadecenone, 3-methyl-5-cyclopentadecenone,3-methylcyclopentadecanone, 4-(1-ethoxyvinyl)-3,3,5,5-tetramethylcyclohexanone, 4-tert-pentylcyclohexanone, 5-cyclohexadecen-1-one, 6,7-dihydro-1,1,2,3,3-pentamethyl-4(5H)-indanone, 9-cycloheptadecen-1-one, cyclopentadecanone, cyclohexadecanone;
- $C_4$-$C_{18}$ cycloaliphatic aldehydes, preferably 2,4-dimethyl-3-cyclohexenecarbaldehyde, 2-methyl-4-(2,2,6-trimethyl-cyclohexen-1-yl)-2-butenal, 4-(4-hydroxy-4-methylpentyl)-3-cyclohexenecarbaldehyde, 4-(4-methyl-3-penten-1-yl)-3-cyclohexenecarbaldehyde;
- $C_4$-$C_{18}$ cycloaliphatic ketones, preferably 1-(3,3-dimethylcyclohexyl)-4-penten-1-one, 1-(5,5-dimethyl-1-cyclohexen-1-yl)-4-penten-1-one, 2,3,8,8-tetramethyl-1,2,3,4,5,6,7,8-octahydro-2-naphthalenyl methyl ketone, methyl-2,6,10-trimethyl-2,5,9-cyclododecatrienyl ketone, tert-butyl(2,4-dimethyl-3-cyclohexen-1-yl)ketone;
- esters of cyclic alcohols in $C_4$-$C_{18}$, preferably 2-tert-butylcyclohexyl acetate, 4-tert-butyl-cyclohexyl acetate, 2-tert-pentylcyclohexyl acetate, 4-tert-pentylcyclohexyl acetate, decahydro-2-naphthyl acetate, 3-pentyltetrahydro-2H-pyran-4-yl acetate, decahydro-2,5,5,8a-tetramethyl-2-naphthyl acetate, 4,7-methano-3a,4,5,6,7,7a-hexahydro-5 or 6-indenyl acetate, 4,7-methano-3a,4,5,6,7,7a-hexahydro-5 or 6-indenyl propionate, 4,7-methano-3a,4,5,6,7,7a-hexahydro-5 or 6-indenyl isobutyrate, 4,7-methanooctahydro-5 or 6-indenyl acetate;
- esters of cycloaliphatic carboxylic acids in $C_4$-$C_{18}$, preferably allyl 3-cyclohexylpropionate, allyl cyclohexyloxyacetate, methyl dihydrojasmonate, methyl jasmonate, methyl 2-hexyl-3-oxocyclopentanecarboxylate, ethyl 2-ethyl-6,6-dimethyl-2-cyclohexenecarboxylate, ethyl 2,3,6,6-tetramethyl-2-cyclohexenecarboxylate, ethyl 2-methyl-1,3-dioxolane-2-acetate;
- $C_4$-$C_{18}$ aromatic hydrocarbons, preferably styrene and diphenylmethane;
- $C_4$-$C_{18}$ araliphatic alcohols, preferably benzyl alcohol, 1-phenylethyl alcohol, 2-phenylethyl alcohol, 3-phenylpropanol, 2-phenylpropanol, 2-phenoxyethanol, 2,2-dimethyl-3-phenylpropanol, 2,2-dimethyl-3-(3-methylphenyl)propanol, 1,1-dimethyl-2-phenylethyl alcohol, 1,1-dimethyl-3-phenylpropanol, 1-ethyl-1-methyl-3-phenylpropanol, 2-methyl-5-phenylpentanol, 3-methyl-5-phenylpentanol, 3-phenyl-2-propen-1-ol, 4-methoxybenzyl alcohol, 1-(4-isopropylphenyl)ethanol;
- esters of araliphatic alcohols in $C_4$-$C_{18}$ and aliphatic carboxylic acids in $C_4$-$C_{18}$, preferably benzyl acetate, benzyl propionate, benzyl isobutyrate, benzyl isovalerate, 2-phenylethyl acetate, 2-phenylethyl propionate, 2-phenylethyl isobutyrate, 2-phenylethyl isovalerate, 1-phenylethyl acetate, α-trichloromethylbenzyl acetate, α,α-dimethylphenylethyl acetate, α,α-dimethylphenylethyl butyrate, cinnamyl acetate, 2-phenoxyethyl isobutyrate, 4-methoxybenzyl acetate;
- $C_2$-$C_{18}$ araliphatic ethers, preferably 2-phenylethyl methyl ether, 2-phenylethyl isoamyl ether, 2-phenylethyl 1-ethoxyethyl ether, phenylacetaldehyde dimethyl acetal, phenylacetaldehyde diethyl acetal, hydratropaldehyde dimethyl acetal, phenylacetaldehyde glycerol acetal, 2,4,6-trimethyl-4-phenyl-1,3-dioxane, 4,4a,5,9b-tetrahydroindeno[1,2-d]-m-dioxin, 4,4a,5,9b-tetrahydro-2,4-dimethylindeno[1,2-d]-m-dioxin;
- $C_4$-$C_{18}$ aromatic and araliphatic aldehydes, preferably benzaldehyde, phenylacetaldehyde, 3-phenylpropanal, hydratropaldehyde, 4-methylbenzaldehyde, 4-methylphenylacetaldehyde, 3-(4-ethylphenyl)-2,2-dimethylpropanal, 2-methyl-3-(4-isopropylphenyl)propanal, 2-methyl-3-(4-tert-butylphenyl)propanal, 3-(4-tert-butylphenyl)propanal, cinnamaldehyde, alpha-butylcinnamaldehyde, alpha-amylcinnamaldehyde, alpha-hexylcinnamaldehyde, 3-methyl-5-phenylpentanal, 4-methoxybenzaldehyde, 4-hydroxy-3-methoxybenzaldehyde, 4-hydroxy-3-ethoxybenzaldehyde, 3,4-methylenedioxybenzaldehyde, 3,4-dimethoxybenzaldehyde, 2-methyl-3-(4-methoxyphenyl)propanal, 2-methyl-3-(4-methylenedioxyphenyl)propanal;
- $C_4$-$C_{18}$ aromatic and araliphatic ketones, preferably acetophenone, 4-methylacetophenone, 4-methoxyacetophenone, 4-tert-butyl-2,6-dimethylacetophenone, 4-phenyl-2-butanone, 4-(4-hydroxyphenyl)-2-butanone, 1-(2-naphthalenyl)ethanone, benzophenone, 1,1,2,3,3,6-hexamethyl-5-indanyl methyl ketone, 6-tert-butyl-1,1-dimethyl-4-indanyl methyl ketone, 1-[2,3-dihydro-1,1,2,6-tetramethyl-3-(1-methylethyl)-1H-5-indenyl]ethanone, 5',6',7',8'-tetrahydro-3',5',5',6',8',8'-hexamethyl-2-acetonaphthone;
- $C_4$-$C_{18}$ aromatic and araliphatic carboxylic acids and esters thereof, preferably phenylacetic acid, methyl benzoate, ethyl benzoate, hexyl benzoate, benzyl benzoate, methyl phenylacetate, ethyl phenylacetate, geranyl phenylacetate, phenylethyl phenylacetate, methyl cinnamate, ethyl cinnamate, benzyl cinnamate, phenylethyl cinnamate, cinnamyl cinnamate, allyl phenoxyacetate, methyl salicylate, isoamyl salicylate, hexyl salicylate, cyclohexyl salicylate, cis-3-hexenyl salicylate, benzyl salicylate, phenylethyl salicylate, methyl 2,4-dihydroxy-3,6-dimethylbenzoate, ethyl 3-phenylglycidate, ethyl 3-methyl-3-phenylglycidate;

- nitrogen-containing aromatic compounds in $C_4$-$C_{18}$, preferably 2,4,6-trinitro-1,3-dimethyl-5-tert-butylbenzene, 3,5-dinitro-2,6-dimethyl-4-tert-butylacetophenone, cinnamonitrile, 5-phenyl-3-methyl-2-pentenenitrile, 5-phenyl-3-methylpentanenitrile, methyl anthranilate, methyl N-methylanthranilate, Schiff bases of methyl anthranilate with 7-hydroxy-3,7-dimethyloctanal, 2-methyl-3-(4-tert-butylphenyl)propanal, 2,4-dimethyl-3-cyclohexene-carbaldehyde, 6-isopropylquinoline, 6-isobutylquinoline, 6-sec-butylquinoline, indole, skatole, 2-methoxy-3-isopropylpyrazine, 2-isobutyl-3-methoxypyrazine;

- phenols, phenyl ethers and phenyl esters, preferably estragole, anethole, eugenol, eugenyl methyl ether, isoeugenol, isoeugenyl methyl ether, thymol, carvacrol, diphenyl ether, beta-naphthyl methyl ether, beta-naphthyl ethyl ether, beta-naphthyl isobutyl ether, 1,4-dimethoxybenzene, eugenyl acetate, 2-methoxy-4-methylphenol, 2-ethoxy-5-(1-propenyl)phenol, p-cresyl phenylacetate;

- heterocyclic compounds in $C_4$-$C_{12}$, preferably 2,5-dimethyl-4-hydroxy-2H-furan-3-one, 2-ethyl-4-hydroxy-5-methyl-2H-furan-3-one, 3-hydroxy-2-methyl-4H-pyran-4-one, 2-ethyl-3-hydroxy-4H-pyran-4-one;

- lactones in $C_4$-$C_{18}$, preferably 1,4-octanolide, 3-methyl-1,4-octanolide, 1,4-nonanolide, 1,4-decanolide, 8-decen-1,4-olide, 1,4-undecanolide, 1,4-dodecanolide, 1,5-decanolide, 1,5-dodecanolide, 1,15-pentadecanolide, cis- and trans-11-pentadecen-1,15-olide, cis- and trans-12-pentadecen-1,15-olide, 1,16-hexadecanolide, 9-hexadecen-1,16-olide, 10-oxa-1,16-hexadecanolide, 11-oxa-1,16-hexadecanolide, 12-oxa-1,16-hexadecanolide, ethylene 1,12-dodecanedioate, ethylene 1,13-tridecanedioate, coumarin, 2,3-dihydrocoumarin, octahydrocoumarin.

In an especially preferred embodiment, if fragrance compounds which can act as Michael donors or acceptors are present in the fragrance composition, the sum of all such compounds makes up at most 5% by weight, relative to the total weight of the fragrance composition.

In one embodiment, the fragrance compounds present in the fragrance do not contain ionizing functional groups, such as sulfonates, sulphates, phosphates or quaternary ammonium ions.

In one embodiment, the fragrance defined above includes one or more support materials, such as solvents or UV stabilizers. Examples of suitable solvents include hydrocarbons such as those sold under the trade name Isopar®; ethers such as those sold under the Dowanol® trade name; benzyl benzoate; isopropyl myristate; dialkyl adipates; dialkyl succinates; dialkyl glutarates such as the dimethyl esters sold under the trade name Flexisolv®; citrate esters, such as triethyl citrate and acetyl tributyl citrate; soybean methyl ester such as ME-S1885 (sold by Peter Cremer NA); diethyl phthalate; diethylene glycol monoethyl ether; 3-methoxy-3-methyl-1-butanol; dipropylene glycol; and isopropylidene glycerol sold under the Augeo® Clean Multi brand name. Examples of UV stabilisers include butyl methoxy dibenzoyl methane; bis ethylhexyloxyphenolmethoxyphenyl triazine; those sold under the Uvinol® trade name such as Uvinul D50 [bis(2,4-dihydroxyphenyl)-methanone], Parsol® 1789 (butyl methoxydibenzoylmethane); and those sold under the Tinogard® trade name.

In one embodiment, the fragrance comprise up to 60% by weight, such as up to 70% by weight, up to 80% by weight, up to 90% by weight or even 100% by weight of fragrance compounds, the remainder (if appropriate) being made up of support materials as defined above.

In one embodiment, the hydrophobic core of the microcapsules comprises at least 70% by weight, such as at least 80% by weight, or at least 90% by weight of fragrance.

In a second aspect, the invention relates to a process for the manufacture of an aqueous dispersion as defined above.

The process for the manufacture of an aqueous dispersion according to the invention may comprise:

- a first step of forming an oil-in-water emulsion wherein the oil phase comprises the constituent(s) of the hydrophobic core, and the oil phase or the aqueous phase comprises at least one $\alpha,\beta$-unsaturated carbonyl compound (i), at least one multifunctional thiol compound (ii), and an optional polymeric stabilizer, according to whether the compounds (i), (ii), and optional polymeric stabilizer, are oil-soluble or water-soluble, and
- optionally, a subsequent step of adding at least one catalyst (iii) preferably selected from ammonia or amine compounds, in particular primary and tertiary amine compounds, nitrogen catalysts others than amine compounds such as amidine compounds, guanidine or arginine, organophosphorous compounds, and water-soluble alkali metal carbonates and hydroxides.

In a first preferred embodiment, the process for the manufacture of an aqueous dispersion according to the invention may comprise the steps of:

a- forming an oil-in-water emulsion wherein the oil phase comprises the constituent(s) of the hydrophobic core, the at least one $\alpha,\beta$-unsaturated carbonyl compound (i), and the at least one multifunctional thiol compound (ii), and the aqueous phase comprises an optional polymeric stabilizer, and

b- optionally, adding the at least one catalyst (iii) preferably selected from ammonia or amine compounds, in particular primary and tertiary amine compounds, nitrogen catalysts others than amine compounds such as amidine compounds, guanidine or arginine, organophosphorous compounds, and water-soluble alkali metal carbonates and hydroxides.

Alternatively, in a second preferred embodiment, the process for the manufacture of an aqueous dispersion according to the invention may comprise the steps of:

a'- adding solid colloidal particles in either the oil phase or the aqueous phase,

b'- forming an oil-in-water emulsion wherein the oil phase comprises the constituent(s) of the hydrophobic core, the at least one $\alpha,\beta$-unsaturated carbonyl compound (i), and the at least one multifunctional thiol compound (ii),

c'- optionally, adding a polymeric stabilizer, and

d'- optionally, adding the at least one catalyst (iii) preferably selected from ammonia or amine compounds, in particular primary and tertiary amine compounds, nitrogen catalysts others than amine compounds such as amidine compounds, guanidine or arginine, organophosphorous compounds, and water-soluble alkali metal carbonates and hydroxides.

The $\alpha,\beta$-unsaturated carbonyl compound (i), the multifunctional thiol compound (ii), and the catalyst (iii), used in the processes of the invention are as defined above for the aqueous dispersion of the invention.

In the preferred processes of the invention, the oil-in-water emulsion may be obtained by mixing, at a temperature ranging from room temperature to about 90°C, and preferably between 60 and 85°C, an oil phase comprising the constituent(s) of the hydrophobic core (i.e. the fragrance), the at least one $\alpha,\beta$-unsaturated carbonyl compound (i), and the at least one multifunctional thiol compound (ii), with the aqueous phase.

In one embodiment, the at least one $\alpha,\beta$-unsaturated carbonyl compound (i) and the at least one multifunctional thiol compound (ii) are used in the process of the invention in amounts such that the molar equivalence ratio of the (meth)acrylate functional groups of the $\alpha,\beta$-unsaturated carbonyl compound(s) (i) to the thiol functional groups of the multifunctional thiol(s) (ii) is in the range from 1:0.6 to 1:1.4, preferably from 1:0.7 to 1:1.3, and more preferably 1:0.8 to 1:1.2, wherein the molar equivalence of each compound (i) and (ii) is calculated as follows:

$$\text{Molar equivalence in a compound} = \text{molar weight of compound/number of functional groups in the compound.}$$

The optional solid colloidal particles (also known as particulate colloids) used in the process defined above in the second preferred embodiment, may have an average particle size comprised between 5 nm and 6 $\mu$m as measured by dynamic light scattering (DLS).

Suitable solid colloidal particles for use within this process include particles of silica, quartz, glass, aluminum (AlO(OH)), lithium magnesium sodium silicates, alumino-silicates (e.g. clays), silicon, copper, tin (SnO), talc, inorganic oxides or hydroxides (e.g. $Fe_2O_3$, $TiO_2$, $Cr_2O_3$), steel, iron, asbestos, nickel, zinc, lead, marble, chalk ($CaCO_3$), gypsum ($CaSO_4$), barytes (e.g. $BaSO_4$), graphite, carbon black, nanocellulose as well as particles extracted from apple juice (INCI name: pyrus malus). In one embodiment, the solid colloidal particles are selected from particles of silica, particles of lithium magnesium sodium silicates, particles of nanocellulose, and particles extracted from apple juice. In a preferred embodiment, the solid colloidal particles are silica particles.

Solid colloidal particles suitable for this process of the invention may or may not be surface modified. Examples of surface modification include chemical treatments to increase or decrease particles hydrophobicity. Alternatively, surface modifying agents can be adsorbed onto particles surface to impart appropriate surface active properties. Alternatively, particles may be modified by means of coupling agents which improve the compatibility between the particles and the microcapsule shell. Techniques to modify particle surfaces are discussed for example in "Nanoparticle Technology handbook" 1st edition, year 2007, Application 41 (pages 593-596) "Surface modification of inorganic nanoparticles by organic functional groups". Modified (as well as non-modified) solid colloidal particles are commercially available.

Examples of suitable colloidal silicas may be dry fumed silicas (such as the range of products marketed under the tradename Aerosil®) or aqueous colloidal silica dispersions (such as the range of products marketed under the tradename Ludox®). Dry silica particles may be fumed silica particles or condensed silica particles. Fumed silicas are particularly adapted for stabilizing emulsions with droplet sizes in the range of 10 $\mu$m to 100 $\mu$m. For larger droplets,

colloidal silicas might be more appropriate. Suitable grades of fumed silica are Aerosil® 200 (a hydrophilic fumed silica with a specific surface area of 200 m$^2$.g$^{-1}$) and Aerosil® R816 having a BET surface area of 190 ± 20 m$^2$.g$^{-1}$ and an average particle size of about 12 nm, both available from Evonik.

The use of solid colloidal particles as presently defined has been found to allow satisfactory and effective microencapsulation of desirably high fragrance loadings while maintaining a suitable quality of the resulting dispersion (e.g., good processability of the dispersion).

In a preferred embodiment, the solid colloidal particles are typically added in an amount ranging from 0.05% to 10% by weight, and preferably from 0.05 to 6% by weight, relative to the weight of the water phase of the oil-in-water emulsion.

In one embodiment, in conjunction with solid colloidal particles, silane coupling agents may be used to increase the compatibility between the polymer formed and the inorganic solid colloidal particles. These silane coupling agents may be (3-mercaptopropyl)trimethoxysilane, (3-mercaptopropyl)ethoxysilane, 3-acrylamidopropyltrimethoxysilane, acryloxymethyltrimethoxysilane, (3-acryloxypropyl)trimethoxysilane, (3-acryloxypropyl)methyldiethoxysilane, 3-acryloxypropyl)methyldimethoxysilane, o-(methacryloxyethyl)-N-(triethoxysilylpropyl)carbamate, methacryloxypropyltrimethoxysilane, methacryloxypropyltriethoxysilane, o-(methacryloxyethyl)-N-(triethoxysilylpropyl)urethane, methacryloxymethyltrimethoxysilane, (methacryloxymethyl)methyl diethoxysilane, (methacryloxymethyl)methyl dimethoxysilane, and mixtures of thereof.

In a preferred embodiment, the silane coupling agent is selected from (3-mercaptopropyl)trimethoxysilane, (3-mercaptopropyl)ethoxysilane, (3-acryloxypropyl)trimethoxysilane, methacryloxypropyltrimethoxysilane, and mixtures thereof.

In an embodiment, the silane coupling agent may be added to the oil phase or aqueous phase, according to whether the silane coupling agent is oil-soluble or water-soluble. In a preferred embodiment, the silane coupling agent is added to the oil phase.

In an embodiment, the silane coupling agent is present in an amount ranging from 50 and 300% by weight, and preferably 100 to 200% by weight, relative to the total weight of colloidal particles.

According to an embodiment, the condensation reaction between the at least one α,β-unsaturated carbonyl compound and (ii) at least one multifunctional thiol compound may be followed by a radical polymerization, to complete the crosslinking of the shell. In this case, the oil phase or the aqueous phase may comprise a free-radical polymerization initiator.

In the preferred processes of the invention, the free-radical polymerization initiator is soluble in the oil phase, and is preferably selected from α,α'-azoisobutyronitrile, 2,2'-azobis(2,4-dimethyl valeronitrile), 2,2'-azobis(4-methoxy-2,4-dimethylvaleronitrile), dimethyl 2,2'-azobis(2-methylpropionate), 1,1'-azo-bis-1-cyclohexanenitrile, dilauroyl peroxide, benzoyl peroxide, tert-butyl peroxydiethylacetate, tert-butyl peroxy-2-ethylhexanoate, di(4-methylbenzoyl)peroxide, 1,1,3,3-tetramethylbutyl peroxy-2-ethylhexanoate, 2,5-dimethyl-2,5-di(2-ethylhexanoylperoxy)hexane, didecanoylperoxide, di(3,5,5-trimethylhexanoyl)peroxide, tert-amyl peroxypivalate, tert-butylpeeoxyneoheptanoate 1,1,3,3-tetramethylbutyl peroxy-pivalate, dicetyl peroxydicarbonate , and mixtures thereof.

In a preferred embodiment, the free-radical polymerization initiator is present in an amount ranging from 0.1 to 5% by weight, and preferably 0.2 to 2% by weight, relative to the total weight of compounds (i) and (ii).

In the processes of the invention, a polymeric stabilizer can also be added to the aqueous phase. Polymeric stabilizers are conventionally used to stabilize oil-in-water emulsions created by mechanical agitation.

In one embodiment, the polymeric stabilizer is selected from:

- cellulose derivatives such as hydroxyethylcellulose, (hydroxypropyl)methylcellulose, carboxymethylcellulose and methylcellulose,
- polyvinylpyrrolidone,
- copolymers of N-vinylpyrrolidone,
- polyvinyl alcohols obtainable by full to partial hydrolyses of polyvinyl acetates,
- polyacrylic and/or polymethacrylic acid,
- copolymers of acrylic acid and methacrylic acid, and esters thereof,
- ionic colloids such as sulphonic-acid-group-containing water-soluble polymers (e.g. 2-acrylamido-2-alkylsulphonic acids and styrene sulphonic acids), and
- mixtures thereof.

In one embodiment, the polymeric stabilizer has a molecular weight greater than about 5,000 g.mol$^{-1}$, preferably greater than about 10,000 g.mol$^{-1}$, more preferably greater than about 50,000 g.mol$^{-1}$. In another embodiment, the polymeric stabilizer has a molecular weight less than about 200,000 g.mol$^{-1}$, preferably less than about 150,000 g.mol$^{-1}$.

In one embodiment, the polymeric stabilizer is a polyvinyl alcohol (PVA), which has preferably a molecular weight as defined above.

In one embodiment, the polymeric stabilizer is present in an amount from about 0.1 to about 10% by weight of the weight of the water phase of the oil-in-water emulsion.

After the emulsion has been stabilized (if required), it is heated to a temperature in the range from about 30°C to about 90°C. The reaction is then left in that temperature range for about 2 hours to about 8 hours, after which time the microcapsules are formed, and the dispersion is allowed to cool to room temperature.

Then, the at least one catalyst (iii), possibly diluted in water, can be added to the aqueous phase of the emulsion during the optional step b- or step d'-.

Optionally, deposition aids can be included to increase deposition or adhesion of the microcapsules to various natural and synthetic surfaces such as various substrates including but not limited to paper, fabric skin, hair, plastic, leather, ceramic, or other surfaces. Deposition aids can include poly (acrylamide-co-diallyldimethylammonium) chloride, poly(diallyldimethylammonium) chloride, polyethylenimine, cationic polyamine, poly[(3-methyl-1-vinylimidazolium chloride)-co-(1-vinylpyrrolidone)], copolymer of acrylic acid and diallyldimethylammonium chloride, cationic guar, guar gum, an organopolysiloxane such as described in US patent application 2015/0030557. Deposition aids can also be selected from poly(meth)acrylate, poly(ethylene-maleic anhydride), polyamine, wax, polyvinylpyrrolidone, polyvinylpyrrolidone co-polymers, polyvinylpyrrolidone-ethyl acrylate, polyvinylpyrrolidone-vinyl acrylate, polyvinylpyrrolidone methylacrylate, polyvinylpyrrolidone-vinyl acetate, polyvinyl acetal, polyvinyl butyral, polysiloxane, poly(propylene maleic anhydride), maleic anhydride derivatives, co-polymers of maleic anhydride derivatives, polyvinyl alcohol, styrenebutadiene latex, gelatin, gum arabic, carboxymethyl cellulose, carboxymethyl hydroxyethyl cellulose, hydroxyethyl cellulose, other modified celluloses, sodium alginate, chitosan, casein, pectin, modified starch, polyvinyl acetal, polyvinyl butyral, polyvinyl methyl ether/maleic anhydride, polyvinyl pyrrolidone and its co polymers, poly(vinyl pyrrolidone/methacrylamidopropyltrimethylammonium chloride), polyvinylpyrrolidone/vinyl acetate, polyvinyl pyrrolidone/dimethylaminoethyl methacrylate, polyvinyl amines, polyvinyl formamides, polyallyl amines and copolymers of polyvinyl amines, polyvinyl formamides, and polyallyl amines and mixtures thereof. In one embodiment, the deposition aid coats the outer surface of the shell of the microcapsule.

The aqueous dispersion of the invention can advantageously be incorporated into a variety of consumer products.

Accordingly, a third aspect of the invention is concerned with a consumer product comprising an aqueous dispersion as defined above. The product may be a non-edible consumer goods product, a household cleaner or laundry product, a personal care product or a cosmetic product, and in particular a laundry product, a personal care product or a cosmetic product.

Unless otherwise indicated, non-edible means non-intended for ingestion by humans or animals. This includes non-food products that may accidentally be swallowed during normal use. Notably, included within the definition of non-edible products are products for dental and oral care, such as toothpastes, mouth washes and lip balms which although not intended for ingestion may nevertheless accidentally enter the gastro-intestinal tract. The formulations and ingredients of liquid household, laundry, personal care and cosmetic products in which the aqueous dispersion of the invention may be used are well known to those skilled in the art, reference may be made to the following works:

- Formulating Detergents and Personal Care Products: A guide to Product Development, by L. Tan Tai Ho, ISBN 1-893997-10-3, published by the AOCS Press;
- A review of: "Liquid Detergents", K-Y. Li, Ed. Surfactant Science Series 67, Marcel Dekker, Inc (ISBN 0-8247-9391-9); and
- Harry's Cosmeticology, 8th Edition, 2000, published by CHS Press (ISBN 0820603724).

Personal care and cosmetic products include products that can be applied to the skin, hair and nails, either as leave on or rinse off product. In the context of the invention "rinse-off" means that the intended product use includes application to skin and/or hair followed by rinsing and/or wiping the product from the skin and/or hair within a few seconds to minutes of the application step. Personal care and cosmetic products include powders, creams, emulsions, lotions, gels and oils for the skin (face, hands, feet etc), tinted bases (liquids and pastes) and liquid impregnated tissues; products for applying and removing make-up from the face and eyes; hair care products including hair tints and bleaches; products for waving, straightening, setting and fixing hair; shaving products including creams, foams mousses and depilatory products; sun bathing products and products for tanning without the sun; deodorant and antiperspirant products.

In one embodiment a personal care or cosmetic product is selected from the group consisting of a shaving aid, a shampoo, a hair-conditioner product, a leave-on-skin-care product, a skin cleansing or washing product (such as a rinse-off skin cleansing or washing product), a moist tissue and a body spray, deodorant or antiperspirant.

Shaving aids specifically include foams, gels, creams and bars (reference can be made for example to US7,069,658, US6,944,952, US6,594,904, US6,182,365, US6,185,822, US6,298,558, US5,113,585).

Shampoos and hair conditioners specifically include two-in-one shampoos and shampoos especially formulated for dry or greasy hair or containing additives such as antidandruff agents. Hair conditioners may be rinse off or leave on hair conditioners also included are hair tonics, bleaches colorants, setting and styling products. Reference can be made for example to US 6,162,423, US 5,968,286, US 5,935,561, US 5,932,203, US 5,837,661, US 5,776,443, US 5,756,436, US 5,661,118, US 5,618,523.

Leave-on-skin-care products comprise skin washing products, moist tissues, body sprays, deodorants and antiperspirants.

Skin washing products specifically include beauty and hygiene bar soaps, shower gels, liquid soaps, body washes, exfoliating gels and pastes (reference can be made for example to US3,697,644, US4,065,398, US4,387,040).

Moist tissues (wipes) specifically include skin cleansing wipes, baby wipes, make-up removal wipes and skin refreshing wipes (reference can be made for example to US4,775,582, WO02/07701, WO2007/069214, WO95/16474).

Body sprays, deodorants and antiperspirants specifically include sticks, liquid roll-on applicators and pressurized sprays.

Household products include hard surface cleaners such as cleaners for floors, solid work surfaces, tiled surfaces, crockery by hand or machine washing and mirrors and glass; and soft furnishing treatments such as liquid cleaners and refresher products such as odour treatment agents as exemplified by Febreze® (P&G). Household cleaners may be in the form of cream cleaners, isotropic liquid cleaners, spray cleaners and pre-moistened surface cleaning wipes (reference can be made for example to WO91/08283, EP743280, WO96/34938, WO01/23510, WO99/28428).

Spray cleaners may be dispensed from a trigger sprayer or aerosol sprayer, as are well known in the art. An aerosol sprayer dispenses the product using propellant pressure, while a trigger sprayer dispenses the product by pumping it under manual actuation. A suitable aerosol dispenser may have a dip tube or bag on valve, according to US 2015/0108163 and/or US 2011/0303766. A suitable trigger sprayer is found in US 8,322,631.

Household products also include freshening composition which may be used in a device for the delivery of a volatile material to the atmosphere or on inanimate surfaces (e.g. fabric surfaces as a fabric refresher).

Household products further include absorbent articles, such as a sanitary product. Preferably said absorbent article comprises an absorbent core, and optionally a backsheet, topsheet, acquisition layer or outer wrapper, wherein the microcapsules of the invention are disposed on the absorbent core or between one or more of the optional layers. The absorbent article can be contained in a polybag or paper carton. The absorbent article may further comprise a lotion. The absorbent article may further comprise one or more adjunct ingredients selected from surfactants, inks, dyes, mineral oils, petrolatum, polysiloxanes, cyclodextrins, clays, silicates, aluminates, vitamins, isoflavones, flavones, metal oxides, short chain organic acids ($C_1$-$C_8$), triglycerides ($C_8$-$C_{22}$), and antioxidants. Laundry products include powdered laundry detergents, detergent tablets and bars, laundry detergent liquids include light duty liquids, heavy duty liquids, concentrated liquid detergents, non or low aqueous laundry liquids and more specialised cleaners for woollen or dark garments; and fabric softeners and pre- and post-wash treatments such as tumble drier sheets, ironing waters and wash additives such as the Lenor Unstoppables™ from P&G, the Dr Beckman™ range of laundry additives from Dr. Beckman, and Vanish™ laundry additives from Reckitt Benckiser.

In one embodiment a laundry product is selected from the group consisting of a fabric softener, a fabric conditioner and a laundry detergent.

Fabric softeners and conditioners specifically include both conventional diluted (e.g. 2% to 8% by weight of softener in the product) liquid active concentration softeners and concentrated (e.g. 10% to 40% by weight of softener in the product) liquid active concentration softeners as well as fabric conditioners which may contain ingredients to protect colors or garment shape and appearance (reference can be made for example to US 6,335,315, US 5,674,832, US 5,759,990, US 5,877,145, US 5,574,179).

Laundry detergents, particularly liquid laundry detergents, specifically include light duty liquid detergents and heavy duty liquid detergents which may be structured multi-phase liquids or isotropic liquids and which may be aqueous or non-aqueous liquids. These liquids may be in bottles or unit dose sachets and they may optionally contain bleaching agents or enzymes (reference can be made for example to US 5,929,022, US 5,916,862, US 5,731,278, US 5,470,507, US 5,466,802, US 5,460,752, US 5,458,810).

The products presently disclosed may contain water and/or surface active material, either as an emulsifier, if the product is an emulsion, or as a detergent active material if the product has some kind of cleaning function. In certain embodiments, the concentration of surface active material in the product will be within the range 0.1-60% by weight; usually the level of surface active material will be 50% by weight or lower; for most products the level of surface active material will be 30% by weight or lower. On the other hand, the level of surface active material will usually be at least 0.1% by weight preferably greater than 1.0%, and more preferably greater than 3.0% by weight. Certain product formulations are water sensitive (e.g. anti-perspirant, deodorant formulations, non-aqueous liquids packaged in water soluble polyvinyl alcohol films), and for these applications it may be desirable to spray dry the microcapsules to remove water, before the microcapsules are incorporated in the product formulation. For products which have a cleaning function, it is likely the level of surface active material will be higher, typically greater than 10% by weight, and preferably greater than 15% by weight. All percentages are expressed by weight over the weight of the product.

Examples of leave-on products containing emulsifiers are: hand and body lotions, make up removing lotions, skin creams, sunscreen products and sunless tanning products and domestic freshener sprays. Also included are articles of manufacture impregnated with liquids, for example pads or wipes impregnated with lotions for make-up application or removal, or to apply sunscreen compounds or sunless tanning agents, for personal cleansing e.g. as moist toilet tissue or baby wipes.

Examples of personal cleansing products containing detergents are: shampoos, body washes, liquid soaps. Some cleaning products may be considered leave on products even though they are used for cleansing if there is no rinsing or further cleaning action after use. Baby wipes are an example, although used for cleaning the liquid deposited on the skin is not removed by rinsing.

The non-rinsed cosmetic, toiletry and personal care compositions described herein can contain various emulsifiers which are useful for emulsifying the various components of the products. Suitable emulsifiers can include any of a wide variety of non-ionic, cationic, anionic, and zwitterionic surface active materials as disclosed in publications such as McCutcheon's, Detergents and Emulsifiers, North American Edition (1986), published by Allured Publishing Corporation and in U.S. 5,011,681, U.S. 4,421,769, U.S. 3,755,560. Amounts of microcapsules dosed into liquid household, laundry, personal care and cosmetic products may vary depending on several aspects such as the desired microcapsule concentration, the proportion of fragrance within the microcapsules and the amount of fragrance necessary to create the olfactory effect desired. After removing all liquid components from a given product (i.e. measured as dry weight) the microcapsules may be present in an amount from 0.01 to 10% by weight, preferably from 0.05% to 2.5% by weight, more preferably from 0.1 to 1.25% by weight over the weight of the product. The dispersion of microcapsules may be incorporated at a suitable stage in the product manufacturing process but usually after any high-shear mixing stage. If liquid at room temperature, it is preferable that the product into which the microcapsules are to be added has a viscosity greater than 20 MPa, for example greater than 100 MPa, or greater than 1,000 MPa, or even greater than 10,000 MPa, when measured at a low (e.g. 10 rpm) spindle speed and at 25°C. If necessary, viscosity can be adjusted through the addition of conventional viscosity modifying agents. Suitable agents as well as equipment and conditions to measure the viscosity of a product are discussed in Rheology Modifiers Handbook, Practical Uses and Applications, by D. D. Braun and M. R. Rosen, published by William Andrew in 1999 (ISBN 978-0-8155-1441-1).

Further embodiments and advantages of the present invention will become apparent to a skilled reader in light of the examples provided below.

**Examples:**

Capsule particle size measurement:

Median volume diameter and span were measured with a laser diffraction/scattering particle size distribution analyzer (trade name: LA 950V2, manufactured by Horiba, Ltd.)). The dispersant was 18 MΩ water. Several droplets of the emulsion or the capsule dispersion were added into the flow cell unit until an acceptable level of laser light obscuration was achieved and triplicate measurements were then immediately performed. For the calculation of the particle size measurement, the refractive indexes were set at 1.33 (for the water dispersant) and 1.47 (for the fragrances and the capsules).The median capsule diameter was measured as a particle size of 50% frequency (median size) on a volumetric basis. The span was calculated according to the following formula:

$$Span = \frac{D(v;0.9) - D(v;0.1)}{D(v;0.5)}$$

in which D(v; 0.9) is the particle size for 90% of the microcapsules by volume, D(v; 0.1) is the particle size for 10% of the microcapsules by volume and D(v; 0.5) is the median volume microcapsule size as previously defined.

Solid content measurement method:

Approximately 2.5 g of aqueous dispersion were accurately weighted in an aluminium weighing dish and dried during four hours at 105°C in order to remove water. The weight of the dry sample was then determined at room temperature and compared to the weight of the dispersion.

Biodegradability:

The following standards can be used to test the biodegradability of the polymer wall of the microcapsules of the invention: OECD 301 B, OECD 301 F, OECD 311, ISO 14852:2018, ASTM D5338, or ISO 14855. The polymer wall of the microcapsules of the invention display a biodegradation rate of at least 5%, such as at least 10%, such as at least 20%, or at least 30%, after 28 days, when tested according to standard OECD 301F.

**Example 1: Composition of fragrance A (wt%)**

| | |
|---|---|
| Verdox (CAS N° 88 41 5) | 38.50 |
| Isobornyl acetate (CAS N° 125-12-2) | 25.00 |
| Dimethyl benzyl carbinyl acetate (CAS N° 151-05-3) | 14.00 |
| 2,4-ivy carbaldehyde (CAS N° 68039-49-6, 144046-32-2) | 6.50 |
| 2-methyl undecanal (CAS N° 110-41-8) | 4.50 |
| Ethyl 2-methylbutyrate (CAS N° 7452-79-1) | 4.50 |
| Ethyl-2-methylpentanoate (CAS N° 39255-32-8) | 3.00 |
| 2-(2-(4-methyl-3-cyclohexenyl-1-yl)propyl)cyclopentanone (CAS N° 95962-14-4) | 2.50 |
| Delta damascone (CAS N° 57378-68-4) | 1.50 |

**Example 2: Synthesis of an aqueous dispersion of microcapsules according to the invention**

*Preparation of reactants:*

A 10% PVA [poly(vinyl alcohol)] aqueous solution was prepared in advance by dissolving Selvol® 823, hydrolyzed to 87-89% (Sekisui), in water.

*Preparation of aqueous dispersion of microcapsules:*

An oil phase was prepared by mixing 85.0 g of isobornyl acetate, 10.95 g of trimethylolpropane triacrylate and 18 g of pentaerythritol tetrakis(3-mercaptopropionate). An aqueous phase was prepared by mixing 40 g of the 10% PVA aqueous solution previously prepared and 140 g of water.
The aqueous phase and the oil phase were placed into a 500 mL-batch reactor equipped with a condenser, a thermometer, a nitrogen inlet and a deflocculating blade (diameter 4 cm). The mixture was stirred at 900 rpm. After 30 minutes, 0.217 g of hexylamine diluted in 20 g of water were added. Nitrogen was bubbled through the mixture. After a period of time T1, the mixture was heated to 60°C within 40 minutes and kept at this temperature for a period of time T2. Finally, the resultant aqueous dispersion of microcapsules was cooled to room temperature within 40 minutes. The volume median diameter (D(v, 0.5)) and the span of the resultant aqueous dispersion of microcapsules were determined by laser diffraction, and the solid content of the aqueous dispersion was measured.

Results:

| Sample number | Period T1 (hours) | Period T2 (hours) | Median volume diameter (D(v, 0.5)) - $\mu$m (Span) | Dry content (%) |
|---|---|---|---|---|
| 1 | 3 | 1 | 65.2 (0.87) | 37.2 |
| 2 | 1 | 3 | 60.5 (0.95) | 37.5 |

**Example 3: Synthesis of an aqueous dispersion of microcapsules according to the invention**

*Preparation of reactants:*

A 10% PVA [poly(vinyl alcohol)] aqueous solution was prepared in advance by dissolving Selvol® 823, hydrolyzed to 87-89% (Sekisui), in water.

*Preparation of aqueous dispersion of microcapsules:*

An oil phase was prepared by mixing 85.0 g of isobornyl acetate, 10.95 g of trimethylolpropane triacrylate, 18 g of pentaerythritol tetrakis(3-mercaptopropionate) and 0.2 g of 2,2'-azobis(2,4-dimethylvaleronitrile). The mixture is stirred in order to obtain a monophasic, homogeneous and transparent phase. An aqueous phase was prepared by mixing 40 g of the 10% PVA aqueous solution previously prepared and 140 g of water. The aqueous phase and the oil phase were placed into a 500 mL-batch reactor equipped with a condenser, a thermometer, a nitrogen inlet and a deflocculating blade (diameter 4 cm). The mixture was stirred at 900 rpm. After 30 minutes, 0.217 g of hexylamine diluted in 20 g of water were added. Nitrogen was bubbled through the mixture. After one hour, the mixture was heated to 60°C within 40 minutes and kept at this temperature for three hours. Finally, the resultant aqueous dispersion of microcapsules was cooled to room temperature within 40 minutes. The volume median diameter (D(v, 0.5)) and the span of the resultant aqueous dispersion of microcapsules were determined by laser diffraction, and the solid content of the aqueous dispersion was measured.

Results:

| Sample number | Median volume diameter (D(v, 0.5)) - $\mu$m (Span) | Dry content (%) |
|---|---|---|
| 3 | 63.7 (0.95) | 36.7 |

**Example 4: Synthesis of an aqueous dispersion of microcapsules according to the invention**

*Preparation of reactants:*

A 10% PVA [poly(vinyl alcohol)] aqueous solution was prepared in advance by dissolving Selvol® 823, hydrolyzed to 87-89% (Sekisui), in water.

*Preparation of aqueous dispersion of microcapsules:*

An oil phase was prepared by mixing 85.0 g of Fragrance, 10.95 g of trimethylolpropane triacrylate and 18 g of pentaerythritol tetrakis(3-mercaptopropionate).

A dispersion of silica in water was prepared separately by stirring 0.7 g of Aerosil® R816 silica and 160 g of water containing 100 mg.L$^{-1}$ of sodium bicarbonate: during 5 minutes using a stirrer bar and at 10,000 rpm during 30 seconds using a high-shear mixer (e.g. Ystral X 10/20 E3-1050 W equipped with a Dispermix head of diameter 40/54 mm).

The oil phase and the dispersion of silica in water were then stirred together at 10,000 rpm for one minute using a high-shear mixer (e.g. Ystral X 10/20 E3-1050 W equipped with a Dispermix head of diameter 40/54 mm). This emulsion was placed into a sealed 500 mL-batch reactor equipped with a condenser, a thermometer, a bottom outlet valve and an anchor stirrer. During all the process, the mixture was stirred at 250 rpm. 37.8 g of the 10% PVA aqueous solution previously prepared were added. After 10 minutes, 0.214 g of hexylamine diluted in 10 g of water was added. Nitrogen was bubbled through the mixture. After one hour, the mixture was heated to 60°C within 40 minutes and kept at this temperature for three hours. Then, the mixture was heated to 70°C within 10 minutes and kept at this temperature for one hour.

Finally, the resultant aqueous dispersion of microcapsules was cooled to the temperature of 40°C within 40 minutes.

The volume median diameter (D(v, 0.5)) and the span of the resultant aqueous dispersion of microcapsules were determined by laser diffraction, and the solid content of the aqueous dispersion was measured.

Results:

| Sample number | Fragrance | Median volume diameter (D(v, 0.5)) - $\mu$m (Span) | | Dry content (%) |
|---|---|---|---|---|
| | | Emulsion | Final microcapsule dispersion | |
| 4 | Isobornyl acetate | 27.43 (0.63) | 30.05 (0.61) | 35.4 |

(continued)

| Sample number | Fragrance | Median volume diameter (D(v, 0.5)) - μm (Span) | | Dry content (%) |
|---|---|---|---|---|
| | | Emulsion | Final microcapsule dispersion | |
| 5 | Fragrance A | 30.5 (0.63) | 28.0 (0.52) | 34.0 |

**Example 5: Synthesis of an aqueous dispersion of microcapsules according to the invention**

*Preparation of reactants:*

A 10% PVA [poly(vinyl alcohol)] aqueous solution was prepared in advance by dissolving Selvol® 823, hydrolyzed to 87-89% (Sekisui), in water.

*Preparation of aqueous dispersion of microcapsules:*

An oil phase was prepared by mixing 85.0 g of Fragrance, 10.95 g of trimethylolpropane triacrylate, 18 g of pentaerythritol tetrakis(3-mercaptopropionate) and 0.2 g of 2,2'-azobis(2,4-dimethylvaleronitrile). The mixture is stirred in order to obtain a monophasic, homogeneous and transparent phase. A dispersion of silica in water was prepared separately by stirring 0.7 g of Aerosil® R816 silica and 160 g of water containing 100 mg.L$^{-1}$ of sodium bicarbonate: during 5 minutes using a stirrer bar and at 10,000 rpm during 30 seconds using a high-shear mixer (e.g. Ystral X 10/20 E3-1050 W equipped with a Dispermix head of diameter 40/54 mm).
The oil phase and the dispersion of silica in water were then stirred together at 10,000 rpm for one minute using a high-shear mixer (e.g. Ystral X 10/20 E3-1050 W equipped with a Dispermix head of diameter 40/54 mm). This emulsion was placed into a sealed 500 mL-batch reactor equipped with a condenser, a thermometer, a bottom outlet valve and an anchor stirrer. During all the process, the mixture was stirred at 250 rpm. 38.8 g of the 10% PVA aqueous solution previously prepared were added. After 10 minutes, 0.214 g of hexylamine diluted in 10 g of water was added. Nitrogen was bubbled through the mixture. After one hour, the mixture was heated to 60°C within 40 minutes and kept at this temperature for three hours. Then, the mixture was heated to 70°C within 10 minutes and kept at this temperature for one hour.
Finally, the resultant aqueous dispersion of microcapsules was cooled to the temperature of 40°C within 40 minutes.
The volume median diameter (D(v, 0.5)) and the span of the resultant aqueous dispersion of microcapsules were determined by laser diffraction, and the solid content of the aqueous dispersion was measured.

Results:

| Sample number | Fragrance | Median volume diameter (D(v, 0.5)) - μm (Span) | | Dry content (%) |
|---|---|---|---|---|
| | | Emulsion | Final microcapsule dispersion | |
| 6 | Isobornyl acetate | 29.70 (0.60) | 32.52 (0.69) | 34.7 |
| 7 | Fragrance A | 31.16 (0.64) | 32.95 (0.48) | 34.5 |

**Example 6: Synthesis of an aqueous dispersion of microcapsules according to the invention**

*Preparation of reactants:*

A 10% PVA [poly(vinyl alcohol)] aqueous solution was prepared in advance by dissolving Selvol® 823, hydrolyzed to 87-89% (Sekisui), in water.

*Preparation of aqueous dispersion of microcapsules:*

An oil phase was prepared by mixing 85.0 g of Fragrance, 10.95 g of pentaerythritol triacrylate, and 18 g of pentaerythritol tetrakis(3-mercaptopropionate).

A dispersion of silica in water was prepared separately by stirring 0.7 g of Aerosil® R816 silica and 160 g of water containing 100 mg.L$^{-1}$ of sodium bicarbonate: during 5 minutes using a stirrer bar and at 10,000 rpm during 30 seconds using a high-shear mixer (e.g. Ystral X 10/20 E3-1050 W equipped with a Dispermix head of diameter 40/54 mm).

The oil phase and the dispersion of silica in water were then stirred together at 10,000 rpm for one minute using a high-shear mixer (e.g. Ystral X 10/20 E3-1050 W equipped with a Dispermix head of diameter 40/54 mm). This emulsion was placed into a sealed 500 mL-batch reactor equipped with a condenser, a thermometer, a bottom outlet valve and an anchor stirrer. During all the process, the mixture was stirred at 250 rpm. 37.8 g of the 10% PVA aqueous solution previously prepared were added. After 10 minutes, 0.214 g of hexylamine diluted in 10 g of water was added. Nitrogen was bubbled through the mixture. After one hour, the mixture was heated to 60°C within 40 minutes and kept at this temperature for three hours. Then, the mixture was heated to 70°C within 10 minutes and kept at this temperature for one hour.

Finally, the resultant aqueous dispersion of microcapsules was cooled to the temperature of 40°C within 40 minutes.

The volume median diameter (D(v, 0.5)) and the span of the resultant aqueous dispersion of microcapsules were determined by laser diffraction, and the solid content of the aqueous dispersion was measured.

Results:

| Sample number | Fragrance | Median volume diameter (D(v, 0.5)) - μm (Span) | | Dry content (%) |
|---|---|---|---|---|
| | | Emulsion | Final microcapsule dispersion | |
| 8 | Fragrance A | 27.6 (0.64) | 49.8 (1.09) | 36.3 |

**Example 7: Synthesis of an aqueous dispersion of microcapsules according to the invention**

*Preparation of reactants:*

A 10% PVA [poly(vinyl alcohol)] aqueous solution was prepared in advance by dissolving Selvol® 823, hydrolyzed to 87-89% (Sekisui), in water.

*Preparation of aqueous dispersion of microcapsules:*

An oil phase was prepared by mixing 85.0 g of Fragrance, 10.95 g of pentaerythritol triacrylate, 18 g of pentaerythritol tetrakis(3-mercaptopropionate) and 0.2 g of 2,2'-azobis(2,4-dimethylvaleronitrile). The mixture is stirred in order to obtain a monophasic, homogeneous and transparent phase. A dispersion of silica in water was prepared separately by stirring 0.7 g of Aerosil® R816 silica and 160 g of water containing 100 mg.L$^{-1}$ of sodium bicarbonate: during 5 minutes using a stirrer bar and at 10,000 rpm during 30 seconds using a high-shear mixer (e.g. Ystral X 10/20 E3-1050 W equipped with a Dispermix head of diameter 40/54 mm).

The oil phase and the dispersion of silica in water were then stirred together at 10,000 rpm for one minute using a high-shear mixer (e.g. Ystral X 10/20 E3-1050 W equipped with a Dispermix head of diameter 40/54 mm). This emulsion was placed into a sealed 500 mL-batch reactor equipped with a condenser, a thermometer, a bottom outlet valve and an anchor stirrer. During all the process, the mixture was stirred at 250 rpm. 38.8 g of the 10% PVA aqueous solution previously prepared were added. After 10 minutes, 0.214 g of hexylamine diluted in 10 g of water was added. Nitrogen was bubbled through the mixture. After one hour, the mixture was heated to 60°C within 40 minutes and kept at this temperature for three hours. Then, the mixture was heated to 70°C within 10 minutes and kept at this temperature for one hour.

Finally, the resultant aqueous dispersion of microcapsules was cooled to the temperature of 40°C within 40 minutes.

The volume median diameter (D(v, 0.5)) and the span of the resultant aqueous dispersion of microcapsules were determined by laser diffraction, and the solid content of the aqueous dispersion was measured.

Results:

| Sample number | Fragrance | Median volume diameter (D(v, 0.5)) - $\mu$m (Span) | | Dry content (%) |
|---|---|---|---|---|
| | | Emulsion | Final microcapsule dispersion | |
| 9 | Fragrance A | 27.1 (0.64) | 28.8 (0.64) | 34.8 |

**Example 8: Synthesis of an aqueous dispersion of microcapsules according to the invention**

*Preparation of reactants:*

A 10% PVA [poly(vinyl alcohol)] aqueous solution was prepared in advance by dissolving Selvol® 823, hydrolyzed to 87-89% (Sekisui), in water.

*Preparation of aqueous dispersion of microcapsules:*

An oil phase was prepared by mixing 85.0 g of Fragrance, 10.95 g of trimethylolpropane triacrylate, 18 g of pentaerythritol tetrakis(3-mercaptopropionate) and 1.19 g of (3-mercaptopropyl)trimethoxysilane.

A dispersion of silica in water was prepared separately by stirring 0.7 g of Aerosil® R816 silica and 160 g of water containing 100 mg.L$^{-1}$ of sodium bicarbonate: during 5 minutes using a stirrer bar and at 10,000 rpm during 30 seconds using a high-shear mixer (e.g. Ystral X 10/20 E3-1050 W equipped with a Dispermix head of diameter 40/54 mm).

The oil phase and the dispersion of silica in water were then stirred together at 10,000 rpm for one minute using a high-shear mixer (e.g. Ystral X 10/20 E3-1050 W equipped with a Dispermix head of diameter 40/54 mm). This emulsion was placed into a sealed 500 mL-batch reactor equipped with a condenser, a thermometer, a bottom outlet valve and an anchor stirrer. During all the process, the mixture was stirred at 250 rpm. 37.8 g of the 10% PVA aqueous solution previously prepared were added. After 10 minutes, 0.217 g of hexylamine diluted in 10 g of water was added. Nitrogen was bubbled through the mixture. After one hour, the mixture was heated to 60°C within 40 minutes and kept at this temperature for three hours. Then, the mixture was heated to 70°C within 10 minutes and kept at this temperature for one hour.

Finally, the resultant aqueous dispersion of microcapsules was cooled to the temperature of 40°C within 40 minutes.

The volume median diameter (D(v, 0.5)) and the span of the resultant aqueous dispersion of microcapsules were determined by laser diffraction, and the solid content of the aqueous dispersion was measured.

Results:

| Sample number | Fragrance | Median volume diameter (D(v, 0.5)) - $\mu$m (Span) | | Dry content (%) |
|---|---|---|---|---|
| | | Emulsion | Final microcapsule dispersion | |
| 10 | Fragrance A | 31.9 (0.60) | 35.4 (0.68) | 32.9 |

**Example 9: Olfactory test**

The aqueous dispersion prepared in example 2 was diluted at 0.3% w/w in water. 35 droplets of this dispersion were deposited homogeneously on a cotton terry towel (30 cm * 20 cm, each about 50 g) prewashed with an unperfumed liquid detergent at 90°C. The terry towel was let dried over 6 hours at room conditions. The intensity of the fragrance was then assessed before and after they were rubbed against hands as a blind experiment by a panel of five trained assessors. Scores were given on an interval scale from 0 (non-noticeable scent) to 5 (very strong scent). The average score before and after rubbing is shown in the table below.

Results:

| Sample number | Averaged olfactory performances | |
|---|---|---|
| | Before rubbing | After rubbing |
| 5 | 0.9 | 2.9 |
| 7 | 0.3 | 4.1 |
| 8 | 0.2 | 4.5 |
| 9 | 0.4 | 4.5 |
| 10 | 0.4 | 4.8 |

These results show that the microcapsules of the aqueous dispersion of the invention contain some perfume which is released when the dry microcapsules are rubbed.

**Claims**

1. An aqueous dispersion of microcapsules, said microcapsules comprising a hydrophobic core and a shell, wherein said shell is formed of the reaction product of (i) at least one $\alpha,\beta$-unsaturated carbonyl compound, and (ii) at least one multifunctional thiol compound.

2. The aqueous dispersion of claim 1, wherein the at least one $\alpha,\beta$-unsaturated carbonyl compound (i) is an anhydride or a multifunctional (meth)acrylamide.

3. The aqueous dispersion of claim 1, wherein the at least one $\alpha,\beta$-unsaturated carbonyl compound (i) is a multifunctional (meth)acrylate.

4. The aqueous dispersion of claim 3, wherein the multifunctional (meth)acrylate is an ester of (meth)acrylic acid with a linear or branched, alicyclic, aromatic or heterocyclic ($C_2$-$C_{24}$)alcohol, or an ester of (meth)acrylic acid with a ($C_2$-$C_{24}$)polyethylene glycol.

5. The aqueous dispersion of claim 4, wherein the multifunctional (meth)acrylate is selected from 1,4-butylene glycol diacrylate, ethylene glycol diacrylate, diethylene glycol diacrylate, triethylene glycol diacrylate, neopentylglycol diacrylate, triglycerol diacrylate, 1,6-hexane diol diacrylate, trimethylolpropane triacrylate, trimethylolpropane ethoxylate triacrylate, trimethylolpropane propoxylate triacrylate, pentaerythritol triacrylate, tris-2-hydroxyethyl isocyanurate triacrylate, glycerol triacrylate, pentaerythritol tetraacrylate, ditrimethylolpropane tetraacrylate, dipentaerythritol pentaacrylate, dipentaerythritol hexaacrylate, 1,3-butylene glycol dimethacrylate, 1,4-butylene glycol dimethacrylate, 1,2-propylene glycol dimethacrylate, 1,3-propylene glycol dimethacrylate, ethylene glycol dimethacrylate, diethylene glycol dimethacrylate, glycerol dimethacrylate, 1,6-hexane diol dimethacrylate, bisphenol A dimethacrylate, bisphenol A ethoxylate dimethacrylate, pentaerythritol trimethacrylate, glycerol trimethacrylate, trimethylolpropane trimethacrylate, tris-2-hydroxyethyl isocyanurate trimethacrylate, ethoxylated pentaerythritol tetramethacrylate, tris[2-(acryloyloxy)ethyl]isocyanurate, 2-[4,6-bis(2-propenoyloxyethyl)-1,3,5-triazin-2-yl]ethyl prop-2-enoate, and mixtures thereof.

6. The aqueous dispersion of claim 5, wherein the multifunctional (meth)acrylate is selected from 1,4-butylene glycol diacrylate, ethylene glycol diacrylate, trimethylolpropane triacrylate, pentaerythritol triacrylate, glycerol triacrylate, pentaerythritol tetraacrylate, ditrimethylolpropane tetraacrylate, 1,4-butylene glycol dimethacrylate, ethylene glycol dimethacrylate, trimethylolpropane trimethacrylate, glycerol trimethacrylate, and mixtures thereof.

7. The aqueous dispersion of any one of claims 1 to 6, wherein the at least one multifunctional thiol compound (ii) is selected from ethylene glycol bismercaptoacetate, 1,8-dimercapto-3,6-dioxaoctane, dimercaptodiethyl sulfide, 1,6-hexanedithiol, propane-1,2,3-trithiol, 1,2-bis[(2-mercaptoethyl)thio]-3-mercaptopropane, tetrakis(7-mercapto-2,5-dithiaheptyl]methane, trimethylolpropane tris(2-mercaptoacetate), pentaerythritol tetrakis(2-mercaptoacetate), ethylene glycol bis(3-mercaptopropionate), dipentaerythritol hexakis(3-mercaptopropionate), 1,4-butanediol bis(3-mercaptopropionate), tris[2-(3-mercaptopropionyloxy)ethyl]isocyanurate, tetraethylene glycol bis(3-mercaptopropionate), trimethylolethane trimercaptoacetate, 1,4-butanediol bismercaptoacetate, trithiocyanuric acid, pentaerythritol tetrakis(3-mercaptopropionate), trimethylolpropane tris(3-mercaptopropionate), trimethylolpropane tris(3-mercaptobutyrate), 1,4-butanediol bis(3-mercaptobutyrate), poly-2-mercaptoacetate, benzene-1,2-dithiol, 1,4-butanedithiol, 4,4'-biphenyldithiol, benzene-1,4-dithiol, toluene-3,4-dithiol, 1,4-dithiothreitol, 1,3,4-thiadiazole-2,5-dithiol, 1,3,5-benzenetrithiol, 4,4'-bis(mercaptomethylbiphenyl), 1,3,5-tris(3-mercaptobutyryloxyethyl)-1,3,5-triazine-2,4,6(1H,3H,5H)-trione, 3,7-dithia-1,9-nonanedithiol, and mixtures thereof.

8. The aqueous dispersion of claim 7, wherein the at least one multifunctional thiol compound (ii) is selected from ethylene glycol bismercaptoacetate, trimethylolpropane tris(3-mercaptopropionate), pentaerythritol tetrakis(3-mercaptopropionate), 1,8-dimercapto-3,6-dioxaoctane, and mixtures thereof, and preferably ethylene glycol bismercaptoacetate, trimethylolpropane tris(3-mercaptopropionate), pentaerythritol tetrakis(3-mercaptopropionate), and mixtures thereof.

9. The aqueous dispersion of any one of claims 1 to 8, wherein the shell is formed in presence of at least one catalyst (iii), preferably selected from ammonia or amine compounds, in particular primary and tertiary amine compounds, nitrogen catalysts others than amine compounds such as amidine compounds, guanidine or arginine, organophosphorous compounds, and water-soluble alkali metal carbonates and hydroxides.

10. The aqueous dispersion of claim 9, wherein the at least one catalyst (iii) is selected from hexylamine, octylamine, pentylamine, diethylamine, triethylamine, diethylene triamine, tetraethylenepentamine, triethylene tetramine, pentaethylene hexamine, lysine, 3-aminopropyl triethoxysilane, 2-amino-2-methyl-1-propanol, 1,8-diazabicyclo[5.4.0]undec-7-ene, 1,5-diazabicyclo[4.3.0]non-5-ene, N,N-dimethylbenzylamine, dimethylphenylphosphine, and mixtures thereof.

11. The aqueous dispersion of claim 9 or claim 10, wherein the at least one catalyst (iii) is an amine compound selected from primary n-($C_2$-$C_{16}$)monoalkylamine compounds or tertiary amine compounds in $C_2$-$C_{24}$.

12. The aqueous dispersion of claim 11, wherein the at least one catalyst (iii) is a primary n-($C_2$-$C_{16}$)monoalkylamine compound, preferably selected from hexylamine, octylamine, pentylamine, and mixtures thereof.

13. The aqueous dispersion of claim 12, wherein the at least one catalyst (iii) is hexylamine.

14. The aqueous dispersion of any one of claims 1 to 13, wherein the amount of $\alpha,\beta$-unsaturated carbonyl compound (i) ranges from 20 and 70% by weight, and preferably from 30 and 60% by weight, relative to the total weight of compounds (i) and (ii).

15. The aqueous dispersion of any one of claims 1 to 14, wherein the amount of multifunctional thiol compounds (ii) ranges from 30 and 80% by weight, and preferably from 40 and 70% by weight, relative to the total weight of compounds (i) and (ii).

16. The aqueous dispersion of any one of claims 1 to 15, wherein the at least one catalyst (iii) is present in an amount ranging from 0.1 to 5% by weight, and preferably from 0.5 to 3% by weight, relative to the total weight of compounds (i) and (ii).

17. The aqueous dispersion of any one of claims 1 to 16, wherein the hydrophobic core comprises a fragrance.

18. A process for the manufacture of an aqueous dispersion according to any one of claims 1 to 17, comprising the steps of:

  - a first step of forming an oil-in-water emulsion wherein the oil phase comprises the constituent(s) of the hydrophobic core, and the oil phase or the aqueous phase comprises at least one $\alpha,\beta$-unsaturated carbonyl compound (i), at least one multifunctional thiol compound (ii), and an optional polymeric stabilizer, according to whether the compounds (i), (ii), and optional polymeric stabilizer, are oil-soluble or water-soluble, and
  - optionally, a subsequent step of adding at least one catalyst (iii) preferably selected from ammonia or amine compounds, in particular primary and tertiary amine compounds, nitrogen catalysts others than amine compounds such as amidine compounds, guanidine or arginine, organophosphorous compounds, and water-soluble alkali metal carbonates and hydroxides.

19. The process of claim 18, comprising the steps of:

a- forming an oil-in-water emulsion wherein the oil phase comprises the constituent(s) of the hydrophobic core, the at least one $\alpha,\beta$-unsaturated carbonyl compound (i), and the at least one multifunctional thiol compound (ii), and the aqueous phase comprises an optional polymeric stabilizer, and

b- optionally, adding the at least one catalyst (iii) preferably selected from ammonia or amine compounds, in particular primary and tertiary amine compounds, nitrogen catalysts others than amine compounds such as amidine compounds, guanidine or arginine, organophosphorous compounds, and water-soluble alkali metal carbonates and hydroxides.

20. The process of claim 18, comprising the steps of:

a'- adding solid colloidal particles in either the oil phase or the aqueous phase,

b'- forming an oil-in-water emulsion wherein the oil phase comprises the constituent(s) of the hydrophobic core, the at least one $\alpha,\beta$-unsaturated carbonyl compound, and the at least one multifunctional thiol compound (ii),

c'- optionally, adding a polymeric stabilizer, and

d'- optionally, adding the at least one catalyst (iii) preferably selected from amine compounds, in particular primary and tertiary amine compounds, nitrogen catalysts others than amine compounds such as amidine compounds, guanidine or arginine, organophosphorous compounds, and water-soluble alkali metal carbonates and hydroxides.

21. The process of any of claims 18 to 20, wherein the oil phase of the oil-in-water emulsion comprises a free-radical polymerization initiator, preferably selected from $\alpha,\alpha'$-azoisobutyronitrile, 2,2'-azobis(2,4-dimethyl valeronitrile), 2,2'-azobis(4-methoxy-2,4-dimethylvaleronitrile), dimethyl 2,2'-azobis(2-methylpropionate), 1,1'-azo-bis-1-cyclohexanenitrile, dilauroyl peroxide, benzoyl peroxide, tert-butyl peroxydiethylacetate, tert-butyl peroxy-2-ethylhexanoate, di(4-methylbenzoyl)peroxide, 1,1,3,3-tetramethylbutyl peroxy-2-ethylhexanoate, 2,5-dimethyl-2,5-di(2-ethylhexanoylperoxy)hexane, didecanoylperoxide, di(3,5,5-trimethylhexanoyl)peroxide, tert-amyl peroxypivalate, tert-butylpeeoxyneoheptanoate 1,1,3,3-tetramethylbutyl peroxy-pivalate, dicetyl peroxydicarbonate, and mixtures thereof.

22. The process of any one of claims 18 to 21, wherein the polymeric stabilizer has a molecular weight from about 5,000 g.mol$^{-1}$ to about 200,000 g.mol$^{-1}$.

23. A consumer product comprising an aqueous dispersion according to any one of claims 1 to 17.

24. The consumer product of claim 23, which is a laundry product, a personal care product or a cosmetic product.

Europäisches
Patentamt

European
Patent Office

Office européen
des brevets

## EUROPEAN SEARCH REPORT

Application Number

EP 20 30 5188

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 108 676 164 A (UNIV ANHUI) 19 October 2018 (2018-10-19) * example 1 * | 1-24 | INV. B01J13/18 A61K8/11 A61Q19/10 C11D3/50 C11D17/00 |
| X | EP 3 556 780 A1 (KAO CORP [JP]) 23 October 2019 (2019-10-23) | 1-17,23, 24 | |
| A | * paragraphs [0129], [0023]; example 1 * | 18-22 | |
| A | CHRISTOPHER O. BOUNDS ET AL: "Preparation and application of microparticles prepared via the primary amine-catalyzed michael addition of a trithiol to a triacrylate", JOURNAL OF POLYMER SCIENCE, PART A: POLYMER CHEMISTRY, vol. 50, no. 3, 14 October 2011 (2011-10-14), pages 409-422, XP055715115, US ISSN: 0887-624X, DOI: 10.1002/pola.25032 * the whole document * | 1-24 | |
| A | Abedin Rubaiyet: "Effect of process mixing on the size distribution and mean diameter of the thiol-triacrylate microcapsules", Thesis, 31 May 2014 (2014-05-31), XP055706377, Louisiana State University Retrieved from the Internet: URL:https://digitalcommons.lsu.edu/cgi/viewcontent.cgi?article=4766&context=gradschool_theses [retrieved on 2020-06-18] * the whole document * | 1-24 | TECHNICAL FIELDS SEARCHED (IPC) B01J A61Q C11D A61K |

The present search report has been drawn up for all claims

| Place of search | Date of completion of the search | Examiner |
|---|---|---|
| The Hague | 23 July 2020 | Mc Donnell, Shane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 30 5188

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2020

| Patent document cited in search report | | Publication date | Patent family member(s) | | Publication date |
|---|---|---|---|---|---|
| CN 108676164 | A | 19-10-2018 | NONE | | |
| EP 3556780 | A1 | 23-10-2019 | CN | 110036038 A | 19-07-2019 |
| | | | EP | 3556780 A1 | 23-10-2019 |
| | | | JP | 6360642 B1 | 18-07-2018 |
| | | | JP | WO2018110638 A1 | 13-12-2018 |
| | | | KR | 20190096985 A | 20-08-2019 |
| | | | US | 2019299186 A1 | 03-10-2019 |
| | | | WO | 2018110638 A1 | 21-06-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150030557 A
- US 7069658 B
- US 6944952 B
- US 6594904 B
- US 6182365 B
- US 6185822 B
- US 6298558 B
- US 5113585 A
- US 6162423 A
- US 5968286 A
- US 5935561 A
- US 5932203 A
- US 5837661 A
- US 5776443 A
- US 5756436 A
- US 5661118 A
- US 5618523 A
- US 3697644 A
- US 4065398 A
- US 4387040 A
- US 4775582 A
- WO 0207701 A
- WO 2007069214 A
- WO 9516474 A

- WO 9108283 A
- EP 743280 A
- WO 9634938 A
- WO 0123510 A
- WO 9928428 A
- US 20150108163 A
- US 20110303766 A
- US 8322631 B
- US 6335315 B
- US 5674832 A
- US 5759990 A
- US 5877145 A
- US 5574179 A
- US 5929022 A
- US 5916862 A
- US 5731278 A
- US 5470507 A
- US 5466802 A
- US 5460752 A
- US 5458810 A
- US 5011681 A
- US 4421769 A
- US 3755560 A

**Non-patent literature cited in the description**

- MICROENCAPSULATION: Methods and Industrial Applications. Marcel Dekker, Inc, 1996
- Kirk Othmer's Encyclopaedia of Chemical Technology
- **A. D. MCNAUGHT ; A. WILKINSON.** IUPAC Compendium of Chemical Terminology. Blackwell Scientific Publications, 1997
- IUPAC Nomenclature of Organic Chemistry. Blackwell Scientific Publications, 1993
- **S. ARCTANDER.** *Perfume Flavors and Chemicals,* 1969, vol. I, II
- The Merck Index. Merck & Co., Inc,
- **E. GUENTHER.** The Essential Oils. D. Van Nostrand Company, 1949

- Surface modification of inorganic nanoparticles by organic functional groups. Nanoparticle Technology handbook. 2007, 593-596
- **L. TAN TAI HO.** Formulating Detergents and Personal Care Products: A guide to Product Development. AOCS Press
- Liquid Detergents. Surfactant Science Series 67. Marcel Dekker, Inc
- Harry's Cosmeticology. CHS Press, 2000
- North American Edition. **MCCUTCHEON'S.** Detergents and Emulsifiers. Allured Publishing Corporation, 1986
- **D. D. BRAUN ; M. R. ROSEN.** Rheology Modifiers Handbook, Practical Uses and Applications. William Andrew, 1999